(19) Europäisches Patentamt
European Patent Office
Office européen des brevets

(11) **EP 2 852 370 B1**

(12) **EUROPEAN PATENT SPECIFICATION**

(45) Date of publication and mention
of the grant of the patent:
**17.10.2018 Bulletin 2018/42**

(21) Application number: **12876904.9**

(22) Date of filing: **14.05.2012**

(51) Int Cl.:
*A61K 8/73* *(2006.01)*      *A61K 8/81* *(2006.01)*
*A61K 8/39* *(2006.01)*      *A61K 8/60* *(2006.01)*
*A61Q 19/00* *(2006.01)*      *A61K 8/37* *(2006.01)*

(86) International application number:
**PCT/CN2012/075439**

(87) International publication number:
**WO 2013/170416 (21.11.2013 Gazette 2013/47)**

(54) **RADIANCE COMPOSITIONS AND METHODS OF USE**

ZUSAMMENSETZUNGEN MIT STRAHLENDER WIRKUNG UND VERFAHREN ZUR
VERWENDUNG

COMPOSITIONS CONFÉRANT DE L'ÉCLAT ET LEURS PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.04.2015 Bulletin 2015/14**

(73) Proprietor: **Johnson & Johnson Consumer Inc.
Skillman, NJ 08558 (US)**

(72) Inventors:
• **HUANG, Samuel**
  **Shanghai 200245 (CN)**
• **ZENG, Lydia**
  **Shanghai 200245 (CN)**
• **GU, Amigo**
  **Shanghai 200245 (CN)**
• **ZHANG, Linda**
  **Shanghai 200245 (CN)**
• **WU, Jiang**
  **Shanghai 200245 (CN)**

(74) Representative: **Carpmaels & Ransford LLP
One Southampton Row
London WC1B 5HA (GB)**

(56) References cited:
WO-A2-2006/068717      CN-A- 1 658 822
US-A1- 2004 223 928      US-A1- 2007 031 354
US-A1- 2008 057 138

**Description**

FIELD OF INVENTION

The present invention relates to compositions for use on skin. More specifically it relates to compositions providing a radiance effect and their use on skin.

DESCRIPTION OF RELATED ART

US 2008057138 A1 concerns topical creams which retard sun exposure damage to skin and restore skin health.
WO 2006068717 A1 concerns compositions and methods for imparting a long-wear and glossy effect to skin, lips, eyelashes, hair, and/or fingernails and toenails.
A wide variety of compositions intended for use in improving the appearance of the skin are known, including compositions for improving the radiance of the skin. Many of such compositions use emollient oil materials having a relatively high refractive index to increase radiance on the skin. Applicants have recognized the need for compositions, including new compositions comprising relatively high refractive oil materials, that provide improved gloss/radiance to skin.

BRIEF DESCRIPTION OF DRAWING

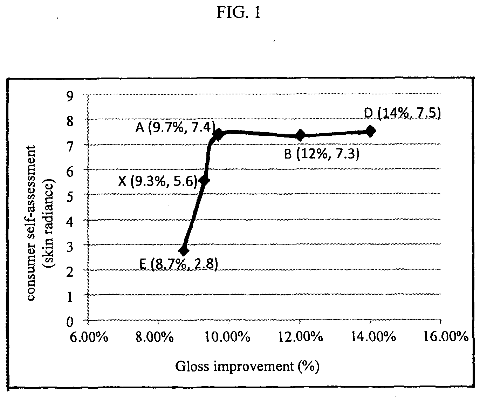

**Fig. 1** is a graphical depiction of the correlation of the consumer perceived skin radiance relative to the percent gloss improvement to the skin associated with certain compositions.

SUMMARY OF THE INVENTION

Applicants have discovered and demonstrated that the claimed compositions provide a significant and unexpected increase in gloss/radiance when used on skin. In particular, Applicants have measured, via the Gloss Determination as described herein, the percent change in gloss on skin (Gloss Improvement %) associated with compositions of the claimed invention as compared to comparable compositions which have only two or fewer of the required materials, or similar materials in different ratios. As shown, for Example, in Tables 1-3, compositions of the claimed invention have a Gloss Improvement % of greater than 9.5% (and even greater in preferred embodiments), whereas certain comparable compositions have a Gloss Improvement of 9.3 or less. Applicants have also recognized a significant difference in the consumer perceived skin radiance associated with compounds having a Gloss Improvement % of 9.5% or greater as compared to compositions having a lower Gloss Improvement %.
Accordingly, in one aspect, the present invention provides compositions comprising a gloss agent selected from the group consisting of ester oils, hydrocarbon oils, silicone oils, natural oils, and combinations of two or more thereof, and having a refractive index, as measured in accordance with the Standard Test Method ASTM D1218, of 1.4 or greater, an emollient film facilitator selected from the group consisting of polyglyceryl esters, hydrocarbons, silicone oils, natural oils, and combinations of two or more thereof having a viscosity, as measured in accordance with the Standard Test Method ASTM D445, of 0.3 Pa·s (300 cPs) or greater, and a polysaccharide film-forming polymer, wherein the compositions comprise a greater amount, on a weight percent basis, of emollient film facilitator than the gloss agent and wherein the gloss agent and the emollient film facilitator are different materials.
In another aspect, the present invention provides methods of providing radiance to the skin by applying to the skin a composition of the present invention.

DESCRIPTION OF INVENTION

As used herein, unless otherwise specified, all percentages of ingredients in compositions are weight percent of active/solids ingredient based on the total weight of composition.
Gloss agents selected from the group consisting of ester oils, hydrocarbon oils, silicone oils, natural oils, and combinations of two or more thereof are used in accord with the present invention. As used herein, a "gloss agent" refers to a material, preferably an emollient oil material, having a refractive index, as measured in accord with the Standard Test Method ASTM D1218, of 1.4 or greater. In certain embodiments, the gloss agent may have a refractive index of 1.42 or greater, including for example, compositions with a refractive index of 1.46 or greater. Examples of suitable gloss agents include ester oils such as isodecyl neopentanoate, isostearyl isostearate, ethylhexyl benzoate, phenethyl benzoate, PPG-3 benzyl ether myristate, Caprylic/capric triglycerides, isopropyl palmitate, isopropyl isostearate, isopropyl myristate, isostearyl isostearate and the like; hydrocarbon oils, such as hydrogenated polydecene, mineral oil, liquid paraffin, squalane, isododecane, isohexadecane, and the like; silicone oils, such as phenyltrimethicone, trimethyl pentaphenyl trisiloxane, diphenyl dimethicone, diphenylsiloxy phenyl trimethicone, and the like; natural plant oils, such as, olive oil, jojoba oil, castor oil, sweet almond oil, avocado oil, grapeseed oil, wheatgerm oil, and the like; as well as combinations of two or more thereof, and the like. Certain preferred gloss agents include ester oils such as isodecyl neopentanoate, isostearyl isostearate, ethylhexyl benzoate, phenethyl benzoate, PPG-3 benzyl ether myristate, and the like and hydrocarbon oils, such as hydrogenated polydecene, and the like. In certain preferred embodiments, the gloss agent is selected from the group consisting of isodecyl neopentanoate, isostearyl isostearate, ethylhexyl benzoate, phenethyl benzoate, PPG-3 benzyl ether myristate, hydrogenated polydecene, and combinations of two or more thereof.

Emollient film facilitators selected from the group consisting of polyglyceryl esters, hydrocarbons, silicone oils, natural oils, and combinations of two or more thereof are used in accord with the present invention. As used herein an "emollient film facilitator" refers to a material suitable for use on skin that is capable of facilitating the formation of a film comprising a film forming polymer of the present invention. Suitable emollient film facilitators have a viscosity higher than 300 cPs including in certain preferred embodiments, materials having a viscosity higher than 1000 cPs, as measured in accord with Standard Test Method ASTM D445.

Examples of suitable emollient film facilitators include polyglyceryl esters, one preferred type of which is polyglyceryl stearate, such as polyglyceryl-2 tetraisostearate, polyglyceryl-2 diisostearate, polyglyceryl-2 isostearate, polyglyceryl-2 triisostearate, and polyglyceryl-3 diisostearate. Other useful examples of suitable emollient film facilitators include sucrose polysoyate, diisostearyl malate, dipentaerythrityl tri-polyhydroxystearate, and the like; hydrocarbons such as hydrogenated polyisobutene, hydrogenated polydecene , and the like; silicone oils, such as, aminopropyl phenyl trimethicone, dimethicone, cetyl dimethicone, aminodimethicone, and the like; natural oils such as shea butter, mango butter, and the like; as well as combinations of two or more thereof, and the like. In certain preferred embodiments, the emollient film facilitator is selected from the group consisting of polyglyceryl-2 tetraisostearate, polyglyceryl-2 diisostearate, polyglyceryl-2 isostearate, polyglyceryl-2 triisostearate, polyglyceryl-3 diisostearate, sucrose polysoyate, diisostearyl malate, dipentaerythrityl tri-polyhydroxystearate, and combinations of two or more thereof.

Any suitable amounts of gloss agent and/or emollient film facilitator may be used in accord with the present invention. For example, in certain embodiments, the compositions of the present invention comprise from greater than zero to 20% of gloss agent in the composition, more preferably from greater than zero to 15%, more preferably from 0.3 to 12%, more preferably from 1 to 5%, more preferably from 2 to 4%. In certain embodiments, the compositions of the present invention comprise from greater than zero to 20% of gloss agent in the composition, more preferably from 1 to 15%, more preferably from 2 to 12%, more preferably from 5 to 10%. In particularly preferred embodiments, the combined amount of gloss agent and emollient film facilitator in the composition is from 2 to 20%, more preferably from 5 to 15%, more preferably 8 to 12%.

The gloss agent and the emollient film facilitator in the compositions of the present invention are different materials. Furthermore, the compositions of the present invention comprise a greater amount (i.e. higher weight percent in composition) of emollient film facilitator as compared to the gloss agent. In certain preferred embodiments, the weight ratio of emollient film facilitator to gloss agent is greater than 1:1, including from greater than 1:1 to 7:1, from greater than 1:1 to 5:1, and from greater than 1:1 to 3:1. In certain preferred embodiments, the ratio is from 3:1 to 7:1, including from 3:1 to 5:1.

Any of a variety of polysaccharide film-forming polymers are used in accord with the present invention. Examples of suitable film-forming polymers include polysaccharide gums such as iota carrageenan gum, gellan gum, xanthan gum, kappa carrageenan gum, agar, konjac gum, combinations of two or more thereof, and the like. In certain preferred embodiments, the film-forming polymer is selected from the group consisting of iota carrageenan gum, gellan gum, xanthan gum, kappa carrageenan gum, and combinations of two or more thereof. In certain preferred embodiments, the film-forming polymer is selected from the group consisting of iota carrageenan gum, xanthan gum, konjac gum, and combinations of two or more thereof.

Any suitable amounts of film-forming polymer may be used in the present invention. Suitable amounts include from greater than zero to 5%, more preferably from 0.005% to 5%, more preferably from 0.005 to 2%, including from 0.005 to 1% and from 0.1 to 1%.

As noted above and illustrated in the Examples, compositions of the present invention tend to have a Gloss Improvement of 9.5% or greater as measured in accord with the Gloss Determination as described herein. In certain preferred embodiments, the compositions of the invention have a Gloss Improvement % of equal to or greater than 9.7%, more preferably greater than 10%, including compositions with Gloss Improvement % of greater than 11%, greater than 12%, and greater than 13%.

Any of a variety of other materials suitable for use in personal care and/or cosmetic compositions may be optionally used in the compositions of the present invention. Examples of such materials include thickeners, conditioners, humectants, chelating agents, exfoliants, surfactants, and additives which enhance the appearance, feel and fragrance of the compositions, such as colorants, fragrances, preservatives, pH adjusting agents, and the like. Examples of suitable thickening agents include: electrolytes (e.g. Sodium Chloride, Ammonium Chloride, Magnesium Chloride); derivatives of natural polysaccharides (e.g. Hydroxyethylcellulose, Ethyl Hydroxyethylcellulose, Cetyl Hydroxyethylcellulose, Methylcellulose, Hydroxypropylcellulose, Sodium Carboxymethylcellulose, Hydroxypropyl Methylcellulose, Hydroxypropyl Guar, Carboxymethyl Hydroxypropyl Guar, C18-22 Hydroxylalkyl Hydroxypropyl Guar); alkali-swellable emulsion (ASE) polymers (e.g. Acrylates Copolymer, available under the trade name Carbopol® AQUA SF-1 from Noveon Consumer Specialites, Brecksville, OH, and Acrylates Copolymer available under the trade name Aculyn™ 33 from Dow Personal Care, Spring House, PA); hydrophobically-modified alkali-swellable emulsion (HASE) polymers (e.g. Acrylates/Steareth-20 Methacrylate Copolymer, Acrylates/Steareth-20 Methacrylate Crosspolymer, and Acrylates/Ceteth-20 Itaconate Copolymer); hydrophobically-modified acid-swellable emulsion polymers (e.g. Acrylates/Aminoacrylates/C10-30 Alkyl PEG-20 Itaconate Copolymer and Polyacrylate-1 Crosspolymer); hydrophobically-modified acrylate crosspolymers, such as Acrylates C10-30 Alkyl Acrylates Crosspolymer, available under the trade name Carbopol® 1382 from Lubrizol Corp., Brecksville, OH; and hydrophobic non-ethoxylated micellar thickeners (e.g. Glyceryl Oleate, Cocamide MIPA, Lauryl Lactyl Lactate, or Sorbitan Sesquicaprylate).

Any of a variety of skin and/or hair conditioning agents are suitable for use and may be used optionally in this invention. Examples include: cationic surfactants (e.g. Cetrimonium Chloride, Stearamidopropyl Dimethylamine, Distearyldimonium Chloride, Lauryl Methyl Gluceth-10 Hydroxypropyldimonium Chloride); cationic polymers (e.g. cationically-modified polysaccharides, including Polyquaternium-10, Polyquaternium-24, Polyquaternium-67, Starch Hydroxypropyltrimonium Chloride, Guar Hydroxypropyltrimonium Chloride, and Hydroxypropyl Guar Hydroxypropyltrimonium Chloride, and cationic polymers derived from the (co)polymerization of ethylenically-unsaturated cationic monomers with optional hydrophilic monomers, including Polyquaternium-5, Polyquaternium-6, Polyquaternium-7, Polyquaternium-11, Polyquaternium-14, Polyquaternium-15, Polyquaternium-28, Polyquaternium-39, Polyquaternium-44; Polyquaternium-76); silicones and silicone derivatives (e.g. Dimethicone and derivatives thereof, such as alkyl-, polyalkyleneoxy-, cationically-, anionically-modified dimethicone (co)polymers); and emollients (e.g. Caprylic/Capric Triglycerides, Mineral Oil, Petrolatum, Di-PPG-2 Myreth-10 Adipate).

Any of a variety of humectants, which are capable of providing moisturization and conditioning properties to the personal cleansing composition, are suitable for use and may be used in the present invention. Examples of suitable humectants nonexclusively include polyols, such as Glycerin, Propylene Glycol, 1,3-Propanediol, Butylene Glycol, Hexylene Glycol, polyglyceryols (e.g. Polyglycerin-3, Polyglyceryn-6, Polyglycerin-10), polyethylene glycols (PEGs), and polyoxyethylene ethers of $\alpha$-methyl glucose, such as Methyl Gluceth-10 and Methyl Gluceth-20.

Examples of suitable chelating agents include those which are capable of protecting and preserving the compositions of this invention. Preferably, the chelating agent is ethylenediamine tetracetic acid ("EDTA").

Examples of suitable preservatives include, for example, organic acids, parabens (e.g. Methylparaben, Ethylparaben, Propylparaben, Butylparaben, Isobutylparaben), quaternary ammonium species (e.g. Quaternium-15), phenoxyethanol, DMDM hydantoin, Diazolidinyl Urea, Imidazolidinyl Urea, Iodopropynyl Butylcarbamate, Methylisothazolinone, Methylchloroisothizaolinone, Benzyl Alcohol, Caprylyl Glycol, Decylene Glycol, Ethylhexylglycerin, and Gluconolactone, natural preserving extracts, and the like.

Furthermore, compositions of the present invention may also include a skin benefit agent. A skin benefit agent is any element, an ion, a compound (e.g., a synthetic compound or a compound isolated from a natural source) or other chemical moiety in solid (e.g. particulate), liquid, or gaseous state and compound that has a cosmetic or therapeutic effect on the skin. As used herein, the term "benefit agent" includes any active ingredient such as a cosmetic or pharmaceutical, that is to be delivered into and/or onto the skin, hair, mucosa, or teeth at a desired location. Examples of suitable benefit agents include those that provide benefits such as, but not limited to: depigmentation agents; amino acids and their derivatives; antimicrobial agents; allergy inhibitors; anti-acne agents; anti-aging agents including anti-wrinkling agents and benefit agents suitable for treating loss of skin elasticity, uneven skin, blotchiness, and skin tone; tropoelasin promoters and tropoelastin crosslinkers; antiseptics; analgesics; antipruritics; local anesthetics; anti-hair loss agents; hair growth promoting agents; hair growth inhibitor agents, antihistamines; antiinfectives; anti-inflammatory agents; anticholinergics; vasoconstrictors; vasodilators; wound healing promoters; peptides, polypeptides and proteins; deodorants and antiperspirants; medicament agents; skin firming agents, vitamins; skin lightening agents; skin darkening agents; antifungals; depilating agents; counterirritants; enzymes for exfoliation or other functional benefits; enzyme inhibitors; NF$\kappa$B-inhibitors; herbal extracts; flavenoids; sensates and stress-reducing agents; anti-oxidants; hair lighteners; sunscreens; anti-edema agents, neo-collagen enhancers, anti-dandruff/sebhorreic dermatitis/psoriasis agents; keratolytics; and mixtures thereof.

The compositions may be made into a wide variety of product types that include but are not limited to washes, gels, sticks, sprays, solid bars, shampoos, pastes, foams, powders, mousses, shaving creams, wipes, patches, nail lacquers, wound dressing and adhesive bandages, hydrogels, films and make-up such as foundations, mascaras, and lipsticks. These product types may comprise several types of carriers including, but not limited to solutions, emulsions (e.g., water-in-oil, oil-in-water, microemulsions and nanoemulsions), suspensions, gels, and solids. Other carriers include solvents, which can include, but are not limited to water, acetone, alcohols, such as isopropanol and ethanol, ethylene glycol, glycerin, dimethylformamide, tertrahydrofuran, dimethylsulfoxide, sorbitol and ethers and ester of sorbitol.

In certain preferred embodiments of the invention, the carrier includes or is water. For example, in certain embodiments, the compositions of the present invention comprise at least 30% water. In more preferred embodiments, compositions of the present invention comprise at least 50% water, more preferably at least 60% water.

In certain preferred embodiments, the gloss agent, emollient film facilitator, and film-forming polymer make up at least 5% by weight or greater of the total solids (e.g. the composition materials without water) in the composition. In certain more preferred compositions, the gloss agent, emollient film facilitator, and film-forming polymer make up at least 25% by weight or greater, more preferably at least 50% by weight or greater of the total solids in the composition. By way of illustration, applicants refer to Example 2 below wherein the gloss agent, emollient film facilitator, and film-forming polymer are present in a combined amount of 10.3 weight percent (2.5+7.5+0.3 weight percent, respectively) and the remaining solids combined are present in an amount of 0.6 weight percent (0.3+0.12+0.08+0.1). Accordingly, the gloss agent, emollient film facilitator, and film-forming polymer make up about 94% by weight of the total solids in the composition of Example 2 (10.3/(10.3+0.6) x 100 = 94%).

The gloss agent, emollient film facilitator, film-forming polymer, and optional other components of the composition may be combined according to the present invention via any conventional methods of combining two or more fluids or solids and/or in accord with the Examples. For example, suitable ingredients may be combined by pouring, mixing, adding dropwise, pipetting, pumping, and the like, one or more of the materials into or with the other in any order using any conventional equipment such as a mechanically stirred propeller, paddle, and the like. In certain embodiments, a general method of making a composition of the present invention comprises combining the water soluble components to form a water based composition and separately combining the oil soluble components to form an oil based composition and then combining the two compositions into one with mixing and allowing the resulting composition to cool down to ambient room temperature. Preferably, the water based composition and the oil based composition are each heated and mixed until all the components in the separate compositions are dissolved into a solution before the two compositions are combined. Homogenizing may be done to mix the final composition well. This way, the composition can form a film that helps to enhance radiance on the skin when it is applied to the skin to improve gloss.

The composition may be impregnated within a substrate (e.g., non-woven fibrous material, a film material, or combinations thereof). The substrate material may be selected to facilitate depositing the material on the skin. The substrate with the composition of the present invention may then be applied on the skin to deposit the composition on the skin.

In certain embodiments, the compositions produced via the present invention are preferably used as or in personal care products and methods of providing radiance to skin, comprising applying to human skin a composition of the present invention. Particularly preferred products are those that are designed to be applied to the skin and not immediately rinsed off. Examples of these "leave-on" products particularly for use on the face, but also including those for the body, hands, feet, and the like. In certain preferred embodiments wherein the composition of the present invention is intended to be a leave on product, it is preferred to limit the amount of surfactants used so as to not interfere with effective film-forming. For example, in such embodiments, the amounts of surfactants are preferably less than 20 weight percent, more preferably less than 15 weight percent, more preferably less than 10 weight percent, and even more preferably less than 5 weight percent of the composition. In certain other embodiments, the composition may be designed for rinse off, such as facial or body or similar cleansing composition. In such rinse-off embodiments, the composition may comprise relatively higher amounts of surfactants, for example, from 10 weight percent to 70 weight percent of the composition.

Accordingly, the invention further provides methods of applying a composition of the claimed invention to the human body, including the face, body, hands, feet, and the like. In certain preferred embodiments, the method is a method of providing radiance to skin, or improving skin radiance comprising applying a composition of the claimed invention to the human face, body, hands, and/or feet, preferably the face. The compositions may be applied to the skin by any suitable means, using ones hands, fingers, a sponge or other porous applicator, by wiping on to the skin, by patting via a pad applicator, among other means. In certain preferred embodiments, the method comprises leaving the composition on the skin without rinsing for at least 5 seconds, more preferably at least 10 seconds, more preferably at least 30 seconds, more preferably at least one minute, more preferably at least 5 minutes.

EXAMPLES

The following Gloss Determination test method was used in the Examples:
A 3.0 cm by 3.0 cm square of human skin on the inner forearm of a test subject was identified as a test area. An initial gloss measurement of the untreated skin in the test area was taken using a Delfin SkinGlossMeter brand glossmeter available from Defin Technologies Ltd. (Kuopio, Finland). Next 30μg of the test composition was applied to the test area via a pipette and the composition was gently rubbed on the skin of the test area for 30 seconds with a finger covered by a rubber glove. The composition was allowed to dry on the test area for 2 minutes and then another gloss measurement was taken using the glossmeter. The Gloss Improvement was then calculated using the following equation:

$$[(\text{Gloss after application} - \text{initial gloss})/\text{initial gloss}] \times 100 = \text{Gloss Improvement (\%)}.$$

Examples 1-12

The following Example compositions were prepared by combining the materials and amounts as indicated in Table 1 as follows: the water soluble components (e.g. Iota carrageenan gum, Acrylates/C10-30 Alkyl Acrylate Crosspolymer, Sodium hydroxide, Methylisothiazolinone, Disodium EDTA) in the amounts indicated in the Tables were added to the water while heating the water phase to 80°C. The oil soluble components (e.g. Hydrogenated polydecene, Polyglyceryl-3 diisostearate) in the amounts indicated were mixed together, heated to 80°C, and poured into the water phase while stirring. If necessary, the resulting mixture was homogenized. The mixture was then cooled to room temperature. The reported amounts of materials in the Tables are active weight % based on total weight of composition.

The Gloss Improvement for each Example was measured as described above and reported in Table 1. For water, q.s. means quantity sufficient to 100%.

Table 1

| Example | 1 | 2 | 3 | 4 | 5 | 6 | 7 | 8 | 9 | 10 | 11 | 12 |
|---|---|---|---|---|---|---|---|---|---|---|---|---|
| Iota carrageenan gum | 0.3 | 0.3 | 0.3 | 0 | 0 | 0 | 0 | 0 | 0.3 | 0.3 | 0.3 | 0.3 |
| Hydrogenated polydecene | 10 | 7.5 | 0 | 10 | 7.5 | 5 | 2.5 | 0 | 5 | 2.5 | 1.7 | 1.25 |
| Polyglyceryl-3 diisostearate | 0 | 2.5 | 10 | 0 | 2.5 | 5 | 7.5 | 10 | 5 | 7.5 | 8.3 | 8.75 |
| Acrylates/C10-30 Alkyl Acrylate Crosspolymer | 0.3 | 0.3 | 0.3 | 0.3 | 0.3 | 0.3 | 0.3 | 0.3 | 0.3 | 0.3 | 0.3 | 0.3 |
| Sodium hydroxide | 0.12 | 0.12 | 0.12 | 0.12 | 0.12 | 0.12 | 0.12 | 0.12 | 0.12 | 0.12 | 0.12 | 0.12 |
| Methylisothiazolinone | 0.08 | 0.08 | 0.08 | 0.08 | 0.08 | 0.08 | 0.08 | 0.08 | 0.08 | 0.08 | 0.08 | 0.05 |
| Disodium EDTA | 0.1 | 0.1 | 0.1 | 0.1 | 0.1 | 0.1 | 0.1 | 0.1 | 0.1 | 0.1 | 0.1 | 0.1 |
| Water | q.s. | q.s. | q.s. | q.s. | q.s. | q.s. | q.s. | q.s. | q.s. | q.s. | q.s. | q.s. |
| Gloss improvement (%) | 8.5 | 8.3 | 9.3 | 8.1 | 8.4 | 7.1 | 7.2 | 6.5 | 10.8 | 14.9 | 10.3 | 11.6 |

Examples 13-17

The following Example compositions (13-17) were prepared as described above for Examples 1-12. The Gloss Improvement for each Example was measured as described above and reported in Table 2.

Table 2

| Example | 13 | 14 | 15 | 16 | 17 |
|---|---|---|---|---|---|
| Gellan Gum | 0.3 | | | | |
| Kappa carrageenan gum | | 0.3 | 0.6 | | |
| Iota carrageenan gum | | | | 0.6 | |
| Xanthan gum | | | | | 0.3 |
| Hydrogenated polydecene | 2.5 | 2.5 | 2.5 | 2.5 | 2.5 |
| Polyglyceryl-3 diisostearate | 7.5 | 7.5 | 7.5 | 7.5 | 7.5 |
| Acrylates/C10-30 Alkyl Acrylate Crosspolymer | 0.3 | 0.3 | 0.3 | 0.3 | 0.3 |
| Sodium hydroxide | 0.12 | 0.12 | 0.12 | 0.12 | 0.12 |
| Methylisothiazolinone | 0.08 | 0.08 | 0.08 | 0.05 | 0.08 |
| Disodium EDTA | 0.1 | 0.1 | 0.1 | 0.1 | 0.1 |
| Water | q.s. | q.s. | q.s. | q.s. | q.s. |
| Gloss Improvement (%) | 10.2 | 10.2 | 11.2 | 15.2 | 12.4 |

Examples 18-22

The following Example compositions with different gloss agents were prepared as described above for the previous Examples. The Gloss Improvement for each Example was measured as described above and reported in Table 3. The refractive index (RI) and viscosity (cPs) of the gloss agents used in the Examples are shown in Table 4.

Table 3

| Example | 10 | 18 | 19 | 20 | 21 | 22 |
|---|---|---|---|---|---|---|
| Iota carrageenan Gum | 0.3 | 0.3 | 0.3 | 0.3 | 0.3 | 0.3 |
| Hydrogenated Polydecene | 2.5 | | | | | |
| Isodecyl Neopentanoate | | 2.5 | | | | |
| Isostearyl Isostearate | | | 2.5 | | | |
| PPG-3 Benzyl ether Myristate | | | | 2.5 | | |
| Ethylhexyl Benzoate | | | | | 2.5 | |
| Phenethyl Benzoate | | | | | | 2.5 |
| Polyglyceryl-3 Diisostearate | 7.5 | 7.5 | 7.5 | 7.5 | 7.5 | 7.5 |
| Acrylates/C10-30 Alkyl Acrylate Crosspolymer | 0.3 | 0.3 | 0.3 | 0.3 | 0.3 | 0.3 |
| Sodium Hydroxide | 0.12 | 0.12 | 0.12 | 0.12 | 0.12 | 0.12 |
| Methylisothiazolinone | 0.08 | 0.08 | 0.08 | 0.08 | 0.08 | 0.05 |
| Disodium EDTA | 0.1 | 0.1 | 0.1 | 0.1 | 0.1 | 0.1 |
| Water | q.s. | q.s. | q.s. | q.s. | q.s. | q.s. |
| Gloss Improvement (%) | 14.9 | 12.1 | 13.2 | 13.8 | 13.2 | 20.9 |

Table 4

|  | RI | Viscosity (cPs) |
|---|---|---|
| Isodecyl Neopentanoate | 1.428 | 4 |
| Isostearyl isostearate | 1.46 | 39.8 |
| Hydrogenated Polydecene | 1.46 | 45 |
| PPG-3 Benzyl ether myristate | 1.4695 | 25.8 |
| Ethylhexyl Benzoate | 1.492 | 25 |
| Phenethyl Benzoate | 1.559 | 40 |

Examples 23-29

The following Example compositions with different emollient film facilitators were prepared as described above for the previous Examples. The Gloss Improvement for each Example was measured as described above and reported in Table 5. The refractive index (RI) and viscosity (cPs) of the emollient film facilitators used in the Examples are shown in Table 6.

Table 5

| Example | 23 | 24 | 25 | 26 | 27 | 28 | 29 |
|---|---|---|---|---|---|---|---|
| Iota carrageenan Gum | 0.3 | 0.3 | 0.3 | 0.3 | 0.3 | 0.3 | 0.3 |
| Hydrogenated Polydecene | 2.5 | 2.5 | 2.5 | 2.5 | 2.5 | 2.5 | 2.5 |
| Polyglyceryl-2 Diisostearate | 7.5 | | | | | | |
| Diisostearyl Malate | | 7.5 | | | | | |
| Polyglyceryl-2 Isostearate | | | 7.5 | | | | |
| Dipentaerythrityl Tri-Polyhydroxystearate | | | | 7.5 | | | |
| Polyglyceryl-2 tetraisostearate | | | | | 7.5 | | |
| Polyglyceryl-2 triisostearate | | | | | | 7.5 | |
| Sucrose Polysoyate | | | | | | | 7.5 |
| Acrylates/C10-30 Alkyl Acrylate Crosspolymer | 0.3 | 0.3 | 0.3 | 0.3 | 0.3 | 0.3 | 0.3 |
| Sodium Hydroxide | 0.12 | 0.12 | 0.12 | 0.12 | 0.12 | 0.12 | 0.12 |
| Methylisothiazolinone | 0.05 | 0.05 | 0.05 | 0.08 | 0.05 | 0.05 | 0.05 |
| Disodium EDTA | 0.1 | 0.1 | 0.1 | 0.1 | 0.1 | 0.1 | 0.1 |
| Water | q.s. | q.s. | q.s. | q.s. | q.s. | q.s. | q.s. |
| Gloss Improvement (%) | 13.7 | 13.8 | 10.2 | 12.2 | 13.7 | 11.5 | 14.3 |

Table 6

|  | RI | Viscosity |
|---|---|---|
| Polyglyceryl-2 tetraisostearate | 1.466 | 369 |
| Sucrose Polysoyate | 1.475 | 419 |
| Polyglyceryl-2 Diisostearate | 1.468 | 1160 |
| Diisostearyl Malate | 1.460 | 5500 |
| Polyglyceryl-2 Isostearate | 1.47 | 6540 |

(continued)

| | RI | Viscosity |
|---|---|---|
| Dipentaerythrityl Tri-Polyhydroxystearate | 1.47 | 15000 |
| Polyglyceryl-2 triisostearate | 1.46 | 38500 |

Examples 30-38

The following Example compositions were prepared as described above for the previous Examples. The Gloss Improvement for each Example was measured as described above and reported in Table 7. Examples 9-12 are also included in Table 7.

Table 7

| Example | 30 | 31 | 32 | 33 | 34 | 35 | 36 | 37 | 38 | 9 | 10 | 11 | 12 |
|---|---|---|---|---|---|---|---|---|---|---|---|---|---|
| Iota carrageenan gum | 0.3 | 0.3 | 0.3 | 0.3 | 0.3 | 0.3 | 0.3 | 0.3 | 0.3 | 0.3 | 0.3 | 0.3 | 0.3 |
| Hydrogenated polydecene | 10 | 5 | 3.3 | 2.5 | 1.25 | 1.7 | | | | 5 | 2.5 | 1.7 | 1.25 |
| Phenethyl Benzoate | | | | | | | 1 | 0.5 | 0.34 | | | | |
| Polyglyceryl-3 diisostearate | 10 | 15 | 16.7 | 2.5 | 3.75 | 3.3 | 1 | 1.5 | 1.66 | 5 | 7.5 | 8.3 | 8.75 |
| Acrylates/C10-30 Alkyl Acrylate Crosspolymer | 0.3 | 0.3 | 0.3 | 0.3 | 0.3 | 0.3 | 0.3 | 0.3 | 0.3 | 0.3 | 0.3 | 0.3 | 0.3 |
| Sodium hydroxide | 0.12 | 0.12 | 0.12 | 0.12 | 0.12 | 0.12 | 0.12 | 0.12 | 0.12 | 0.12 | 0.12 | 0.12 | 0.12 |
| Methylisothiazolinone | 0.08 | 0.08 | 0.08 | 0.08 | 0.08 | 0.05 | 0.05 | 0.05 | 0.05 | 0.05 | 0.05 | 0.05 | 0.05 |
| Disodium EDTA | 0.1 | 0.1 | 0.1 | 0.1 | 0.1 | 0.1 | 0.1 | 0.1 | 0.1 | 0.1 | 0.1 | 0.1 | 0.1 |
| Water | q.s. | q.s. | q.s. | q.s. | q.s. | q.s. | q.s. | q.s. | q.s. | q.s. | q.s. | q.s. | q.s. |
| Gloss Improvement (%) | 6.2 | 10.8 | 8.2 | 9.1 | 10.6 | 9.02 | 6.6 | 8.8 | 10.9 | 10.8 | 14.9 | 10.3 | 11.6 |

Examples 39-40

The following Example compositions were prepared as described above for the previous Examples. The Gloss Improvement was measured as described above and reported in Table 8.

Table 8

| Example | 39 | 40 |
|---|---|---|
| Iota carrageenan gum | 0.05 | 0.005 |
| Hydrogenated polydecene | 2.5 | 2.5 |
| Polyglyceryl-3 diisostearate | 7.5 | 7.5 |
| Acrylates/C10-30 Alkyl Acrylate Crosspolymer | 0.3 | 0.3 |
| Sodium hydroxide | 0.12 | 0.12 |
| Methylisothiazolinone | 0.08 | 0.08 |
| Disodium EDTA | 0.1 | 0.1 |
| Water | q.s. | q.s. |
| Gloss improvement (%) | 12.4 | 11.9 |

Examples 41-42

The following Example compositions were prepared as described above for the previous Examples, except that the materials in the water phase were heated to 90°C prior to mixing with the oil components (it was observed that the Agar or Konjac Gum were not sufficiently soluble in water at lower temperatures). The Gloss Improvement was measured as described above and reported in Table 9.

Table 9

| Example | 41 | 42 |
|---|---|---|
| Agar | 0.3 | |
| Konjac Gum | | 0.3 |
| Hydrogenated polydecene | 2.5 | 2.5 |
| Polyglyceryl-3 diisostearate | 7.5 | 7.5 |
| Acrylates/C10-30 Alkyl Acrylate Crosspolymer | 0.3 | 0.3 |
| Sodium hydroxide | 0.12 | 0.12 |
| Methylisothiazolinone | 0.08 | 0.08 |
| Disodium EDTA | 0.1 | 0.1 |
| Water | q.s. | q.s. |
| Gloss Improvement (%) | 9.7 | 12.8 |

Examples 43-46

The following Example shows the relative correlation between consumer perceived skin gloss and Gloss Improvement %.
Examples 43-46 were prepared as described above for the previous Examples using the materials and amounts as shown in Tables 10-12 below. The Gloss Improvement for each and was measured and is shown in the Tables.

Table 10

| Example | 43 | 44 |
|---|---|---|
| Iota carrageenan Gum | 0.5 | 0.5 |
| Hydrogenated Polydecene | 2.5 | 2.5 |
| Polyglyceryl-2 Diisostearate | 7.5 | |
| Dipentaerythrityl Tri-Polyhydroxystearate | | 7.5 |
| Acrylates/Steareth-20 Methacrylate Copolymer | 2.5 | 2.5 |
| Sodium Hydroxide | 0.13 | 0.13 |
| Methylisothiazolinone | 0.08 | 0.08 |
| Disodium EDTA | 0.05 | 0.05 |
| Water | q.s. | q.s. |
| Gloss Improvement (%) | 14.0 | 12.0 |

Table 11

| Example | 45 |
|---|---|
| Glycerin | 5.0 |
| Disodium EDTA | 0.05 |
| Methyl paraben | 0.15 |
| Propyl paraben | 0.1 |
| Niacinamide | 1.0 |
| Iota carrageenan gum | 0.3 |
| Titanium dioxide | 0.1 |
| Polyacrylamide; C13-14 Isoparaffin; Laureth-7; Water | 1.9 |
| Mineral oil | 5.0 |
| Dimethicone | 1.0 |
| Isopropyl palmitate | 1.5 |
| Diisostearate malate | 1.5 |
| PEG-100 stearate | 0.6 |
| Glyceryl stearate | 0.4 |
| Cetyl alcohol | 0. 3 |
| Stearyl alcohol | 0.2 |
| Cetearyl alcohol; Cetearyl glucoside | 1.5 |
| Dimethicone; Dimethiconol | 1.5 |
| Water; DMDM hydantoin | 0.3 |
| Water | q.s. |
| Gloss Improvement (%) | 9.7 |

Table 12

| Example | 46 |
|---|---|
| Iota carrageenan Gum | 0.3 |
| Phenethyl Benzoate | 1.5 |
| Polyglyceryl-3 Diisostearate | 0.5 |
| Acrylates/C10-30 Alkyl Acrylate Crosspolymer | 0.3 |
| Sodium Hydroxide | 0.12 |
| Methylisothiazolinone | 0.08 |
| Disodium EDTA | 0.1 |
| Water | q.s. |
| Gloss Improvement (%) | 8.7 |

The consumer perceived gloss associated with five different compositions (A, B, D, E, and X, corresponding to Examples 45, 44, 43, 46, and 3 as shown in Table 13) each having a Gloss Improvement value as shown below in Table 13 was determined.

Table 13

| Test Composition | Gloss Improvement % | Consumer Perceived Gloss value |
|---|---|---|
| A (Example 45) | 9.7 | 7.4 |
| B (Example 44) | 12 | 7.3 |
| D (Example 43) | 14 | 7.5 |
| E (Example 46) | 8.7 | 2.8 |
| X (Example 3) | 9.3 | 5.6 |

The consumer perceived gloss was determined as follows:

A 3.0 cm by 3.0 cm square of human skin on the inner forearm of a test subject was identified as a test area. Next 30 μg of the test composition was applied to the test area via a pipette and the composition was gently rubbed on the skin of the test area for 30 seconds with a finger covered by a rubber glove. The composition was allowed to dry on the test area for 2 minutes. Then consumers were asked to rate the gloss level of this area on a 1-10 scale (1 represents lowest gloss level and 10 represents highest gloss level). In total, 18 subjects were used. Final value was calculated by averaging the eighteen ratings for each sample composition.

The resulting consumer perceived gloss (consumer self-assessment) values were plotted relative to the Gloss Improvement values for each of the five tested compositions, and the results are shown in Fig. 1. As illustrated, there is a significant difference in perception associated with compositions having a Gloss Improvement of 9.5% and above, in particular at 9.7% and above, as compared to the compositions having a Gloss Improvement below 9.5%.

## Claims

1. A composition comprising a gloss agent selected from the group consisting of ester oils, hydrocarbon oils, silicone oils, natural oils, and combinations of two or more thereof, and having a refractive index, as measured in accordance with the Standard Test Method ASTM D1218, of 1.4 or greater, an emollient film facilitator selected from the group consisting of polyglyceryl esters, hydrocarbons, silicone oils, natural oils, and combinations of two or more thereof having a viscosity, as measured in accordance with the Standard Test Method ASTM D445, of 0.3 Pa·s (300 cPs) or greater, and a polysaccharide film-forming polymer, wherein the composition comprises a greater amount, on a weight percent basis, of emollient film facilitator than the gloss agent and wherein the gloss agent and the emollient film facilitator are different materials.

2. The composition of claim 1 wherein the gloss agent is selected from the group consisting of isodecyl neopentanoate, isostearyl isostearate, ethylhexyl benzoate, phenethyl benzoate, PPG-3 benzyl ether myristate, hydrogenated polydecene, and combinations of two or more thereof.

3.  The composition of claim 1 wherein the gloss agent has a refractive index of 1.42 or greater.

4.  The composition of claim 1 wherein the emollient film facilitator is selected from the group consisting of polyglyceryl-2 tetraisostearate, polyglyceryl-2 diisostearate, polyglyceryl-2 isostearate, polyglyceryl-2 triisostearate, polyglyceryl-3 diisostearate, sucrose polysoyate, diisostearyl malate, dipentaerythrityl tri-polyhydroxystearate, and combinations of two or more thereof.

5.  The composition of claim 1 wherein the emollient film facilitator has a viscosity of 1 Pa·s (1000 cPs) or greater.

6.  The composition of claim 1 wherein the weight ratio of emollient film facilitator to gloss agent is from greater than 1:1 to 7:1, or from 3:1 to 7:1.

7.  The composition of claim 1 wherein the film-forming polymer is selected from the group consisting of iota carrageenan gum, gellan gum, xanthan gum, kappa carrageenan gum, agar, konjac gum, and combinations of two or more thereof.

8.  The composition of claim 1 wherein the film-forming polymer is selected from the group consisting of iota carrageenan gum, xanthan gum, konjac gum, and combinations of two or more thereof.

9.  The composition of claim 1 having a Gloss Improvement of 9.5% or greater or 10% or greater.

10. The composition of claim 2 wherein the emollient film facilitator is selected from the group consisting of polyglyceryl-2 tetraisostearate, polyglyceryl-2 diisostearate, polyglyceryl-2 isostearate, polyglyceryl-2 triisostearate, polyglyceryl-3 diisostearate, sucrose polysoyate, diisostearyl malate, dipentaerythrityl tri-polyhydroxystearate, and combinations of two or more thereof, and the film-forming polymer is selected from the group consisting of iota carrageenan gum, gellan gum, xanthan gum, kappa carrageenan gum, and combinations of two or more thereof.

11. A product comprising a composition of claim 1 wherein the product is in the form of a personal care wash, gel, stick, spray, solid bar, shampoo, paste, foam, powder, mousse, wipe, patch, film, foundation, mascara, or lipstick.

12. A method of providing radiance to skin comprising applying to human skin a composition of claim 1.

13. A method of providing radiance to skin comprising applying to human skin a composition of claim 10.

14. The method of claim 12 or 13 wherein the applying step comprising applying to skin of the human face.

**Patentansprüche**

1.  Zusammensetzung umfassend ein Glanzmittel ausgewählt aus der Gruppe bestehend aus Esterölen, Kohlenwasserstoffölen, Siliconölen, natürlichen Ölen und Kombinationen von zwei oder mehreren davon und das einen Brechungsindex, wie dem Standardmessverfahren ASTM D1218 entsprechend gemessen, von 1,4 oder mehr aufweisen, einen Emolliensfilmvermittler ausgewählt aus der Gruppe bestehend aus Polyglycerylestern, Kohlenwasserstoffen, Siliconölen, natürlichen Ölen und Kombinationen von zwei oder mehr davon, der eine Viskosität, wie dem Standardmessverfahren ASTM D445 entsprechend gemessen, von 0,3 Pa·s (300 CPs) oder mehr aufweist, und ein Polysaccharidfilm bildendes Polymer, wobei die Zusammensetzung eine höhere Menge, auf einer Gewichtsprozentbasis, von Emolliensfilmvermittler als Glanzmittel umfasst und wobei das Glanzmittel und der Emolliensfilmvermittler verschiedene Materialien sind.

2.  Zusammensetzung nach Anspruch 1, wobei das Glanzmittel aus der Gruppe ausgewählt ist bestehend aus Isodecylneopentanoat, Isostearylisostearat, Ethylhexylbenzoat, Phenethylbenzoat, PPG-3-Benzylethermyristat, hydriertem Polydecen und Kombinationen von zwei oder mehr davon.

3.  Zusammensetzung nach Anspruch 1, wobei das Glanzmittel einen Brechungsindex von 1,42 oder mehr aufweist.

4.  Zusammensetzung nach Anspruch 1, wobei der Emolliensfilmvermittler aus der Gruppe ausgewählt ist bestehend aus Polyglyceryl-2-tetraisostearat, Polyglyceryl-2-diisostearat, Polyglyceryl-2-isostearat, Polyglyceryl-2-triisostearat, Polyglyceryl-3-diisostearat, Sucrosepolysoyat, Diisostearylmaleat, Dipentaerythrittripolyhydroxystearat und Kombinationen von zwei oder mehr davon.

5. Zusammensetzung nach Anspruch 1, wobei der Emolliensfilmvermittler eine Viskosität von 1 Pa·s (1000 CPs) oder mehr aufweist.

6. Zusammensetzung nach Anspruch 1, wobei das Gewichtsverhältnis von Emolliensfilmvermittler zu Glanzmittel von mehr als 1:1 bis 7:1 oder von 3:1 bis 7:1 beträgt.

7. Zusammensetzung nach Anspruch 1, wobei das filmbildende Polymer aus der Gruppe ausgewählt ist bestehend aus Carrageengummi, Gellangummi, Xanthangummi, Kappa-Carrageengummi, Agar, Teufelszungengummi und Kombinationen von zwei oder mehreren davon.

8. Zusammensetzung nach Anspruch 1, wobei das filmbildende Polymer aus der Gruppe ausgewählt ist bestehend aus Iota-Carrageengummi, Xanthangummi, Teufelszungengummi und Kombinationen von zwei oder mehr davon.

9. Zusammensetzung nach Anspruch 1, die eine Glanzverbesserung von 9,5 % oder mehr oder 10 % oder mehr aufweist.

10. Zusammensetzung nach Anspruch 2, wobei der Emolliensfilmvermittler aus der Gruppe ausgewählt ist bestehend aus Polyglyceryl-2-tetraisostearat, Polyglyceryl-2-diisostearat, Polyglyceryl-2-isostearat, Polyglyceryl-2-triisostearat, Polyglyceryl-3-diisostearat, Sucrosepolysoyat, Diisostearylmaleat, Dipentaerythrittripolyhydroxystearat und Kombinationen von zwei oder mehr davon und das filmbildende Polymer aus der Gruppe ausgewählt ist bestehend aus Iota-Carrageengummi, Gellangummi, Xanthangummi, Kappa-Carrageengummi und Kombinationen von zwei oder mehreren davon.

11. Produkt umfassend eine Zusammensetzung nach Anspruch 1, wobei das Produkt in Form eines Körperpflegewaschprodukts, Gels, Stifts, Sprays, festen Stücks, Shampoons, einer Paste, eines Schaums, Puders, Mousse, Wischtuchs, Pflasters, Films, einer Foundation, eines Mascaras oder Lippenstifts vorliegt.

12. Verfahren zum Bereitstellen eines strahlenden Aussehens der Haut, umfassend das Auftragen einer Zusammensetzung nach Anspruch 1 auf die menschliche Haut.

13. Verfahren zum Bereitstellen eines strahlenden Aussehens der Haut umfassend das Auftragen einer Zusammensetzung nach Anspruch 10 auf die menschliche Haut.

14. Verfahren nach Anspruch 12 oder 13, wobei der Auftragsschritt das Auftragen auf die Haut des menschlichen Gesichts umfasst.

**Revendications**

1. Composition comprenant un agent de brillance choisi dans le groupe constitué par les huiles d'esters, les huiles hydrocarbonées, les huiles de silicone, les huiles naturelles, et des combinaisons de deux, ou plus, parmi celles-ci, et ayant un indice de réfraction, tel que mesuré conformément à la Méthode de Test Standard ASTM D1218, de 1,4 ou plus, un facilitateur de film émollient choisi dans le groupe constitué par les esters de polyglycéryle, les hydrocarbures, les huiles de silicone, les huiles naturelles, et des combinaisons de deux, ou plus, parmi ceux-ci, ayant une viscosité, telle que mesurée conformément à la Méthode de Test Standard ASTM D445, de 0,3 Pa.s (300 cPs) ou plus, et un polymère filmogène polysaccharidique, où la composition comprend une quantité supérieure, sur une base de pourcentage pondéral, de facilitateur de film émollient par rapport à l'agent de brillance, et où l'agent de brillance et le facilitateur de film émollient sont des matériaux différents.

2. Composition selon la revendication 1, dans laquelle l'agent de brillance est choisi dans le groupe constitué par le néopentanoate d'isodécyle, l'isostéarate d'isostéaryle, le benzoate d'éthylhexyle, le benzoate de phénéthyle, le myristate d'éther benzylique PPG-3, le polydécène hydrogéné, et des combinaisons de deux, ou plus, parmi ceux-ci.

3. Composition selon la revendication 1, dans laquelle l'agent de brillance possède un indice de réfraction de 1,42 ou plus.

**4.** Composition selon la revendication 1, dans laquelle le facilitateur de film émollient est choisi dans le groupe constitué par le tétraisostéarate de polyglycéryle-2, le diisostéarate de polyglycéryle-2, l'isostéarate de polyglycéryle-2, le triisostéarate de polyglycéryle-2, le diisostéarate de polyglycéryle-3, le polysoyate de saccharose, le malate de diisostéaryle, le tri-polyhydroxystéarate de dipentaérythrityle, et des combinaisons de deux, ou plus, parmi ceux-ci.

**5.** Composition selon la revendication 1, dans laquelle le facilitateur de film émollient possède une viscosité de 1 Pa.s (1000 cPs) ou plus.

**6.** Composition selon la revendication 1, dans laquelle le rapport pondéral du facilitateur de film émollient à l'agent de brillance va de plus de 1:1 à 7:1, ou de 3:1 à 7:1.

**7.** Composition selon la revendication 1, dans laquelle le polymère filmogène est choisi dans le groupe constitué par la gomme d'iota-carraghénane, la gomme gellane, la gomme xanthane, la gomme de kappa-carraghénane, l'agar-agar, la gomme de konjac, et des combinaisons de deux, ou plus, parmi celles-ci.

**8.** Composition selon la revendication 1, dans laquelle le polymère filmogène est choisi dans le groupe constitué par la gomme d'iota-carraghénane, la gomme xanthane, la gomme de konjac, et des combinaisons de deux, ou plus, parmi celles-ci.

**9.** Composition selon la revendication 1, ayant une Amélioration de la Brillance de 9,5% ou plus, ou de 10% ou plus.

**10.** Composition selon la revendication 2, dans laquelle le facilitateur de film émollient est choisi dans le groupe constitué par le tétraisostéarate de polyglycéryle-2, le diisostéarate de polyglycéryle-2, l'isostéarate de polyglycéryle-2, le triisostéarate de polyglycéryle-2, le diisostéarate de polyglycéryle-3, le polysoyate de saccharose, le malate de diisostéaryle, le tri-polyhydroxystéarate de dipentaérythrityle, et des combinaisons de deux, ou plus, parmi ceux-ci, et le polymère filmogène est choisi dans le groupe constitué par la gomme d'iota-carraghénane, la gomme gellane, la gomme xanthane, la gomme de kappa-carraghénane, et des combinaisons de deux, ou plus, parmi celles-ci.

**11.** Produit comprenant une composition selon la revendication 1, où le produit se trouve sous la forme d'une lotion nettoyante de soin personnel, d'un gel, d'un bâtonnet, d'un spray, d'un pain solide, d'un shampooing, d'une pâte, d'un produit expansé, d'une poudre, d'une mousse, d'une lingette, d'un patch, d'un film, d'un fond de teint, d'un mascara ou d'un rouge à lèvres.

**12.** Méthode destinée à apporter de l'éclat à la peau, comprenant l'application à la peau humaine d'une composition selon la revendication 1.

**13.** Méthode destinée à apporter de l'éclat à la peau, comprenant l'application à la peau humaine d'une composition selon la revendication 10.

**14.** Méthode selon la revendication 12 ou 13, dans laquelle l'étape d'application comprenant une application à la peau du visage humain.

FIG. 1

**EP 2 852 370 B1**

**Patent documents cited in the description**

- US 2008057138 A1
- WO 2006068717 A1